# EUROPEAN PATENT APPLICATION

(11) **EP 1 074 633 A1**
(43) Date of publication of application: **07.02.2001**
(21) Application number: 99112943.8
(22) Date of filing: 05.07.1999
(51) Int. Cl.: C12Q 1/68, G01N 27/447

(54) **Nucleotides for cycle sequencing of gc-rich templates**

(71) Applicant: LION Bioscience AG, 69120 Heidelberg (DE)
(72) Inventor: Motz, Michael, 69123 Heidelberg (DE); Voss, Hartmut, Dr., 69221 Dossenheim (DE)
(74) Representative: Goddar, Heinz J., Dr.

(57) **Abstract**

The present invention relates to a mixture comprising 7-deaza dGTP or a functional analogue thereof and dITP. The invention further relates to a method for sequencing and/or amplification of a nucleic acid sequence comprising the steps of preparing a sequencing mixture comprising, among others, 7-deaza dGTP or a functional analogue thereof and dITP, further (NH₄)₂SO₄, a nucleic acid sequence to be sequenced and/or amplified, and some enzymatic activity for incorporating dNTPs and/or ddNTPs or derivatives thereof.

## Description

The present invention relates to a mixture comprising certain nucleotides, a combination comprising said mixture, a sequencing mixture, use of said mixture, a method for sequencing and amplifying nucleic acid sequences and a sequence gel.

Modern biology and biotechnology in its broadest sense strongly rely on the knowledge of DNA sequences. Among others, the human genome project started in the mid-nineties requires the establishment of high throughput sequencing techniques which are capable of providing sequence data with a very high level of reliability. Today, one of the most widely used sequencing techniques is the Sanger DNA sequencing.

One common problem in Sanger DNA-sequencing is that certain DNA fragments show anomalies in their migration behavior during gel-electrophoresis through the polyacrylamide gel. This leads to band compression and uncertain base calling.

This phenomenon is caused by the ability of single-stranded DNA to form base-paired hairpin structures in the DNA-seqeunce (Sanger F., Nicklen, S. and Coulson, A. R.. DNA Sequencing with chain-terminating Inhibitors. *Proc. Natl. Acad. Sci. U.S.A.* **74**, (1977), 5463-5467, Mills D.R. and Kramer, F.R., Structure-independent Nucleotide Sequnce Analysis. *Proc. Natl. Acad. Sci. U.S:A.* **76**, (1976), 2232-2235, Mizusawa S., Nishimura, S. and Seela, F., Improvement of the dideoxy chain termination method of DNA sequencing by use of deoxy-7-deazaguanosine triphosphate in place of dGTP. *Nucleic Acids Res.* 14, (1986), 1319-1324.). This generally is a result of the ability of guanine residues to undergo intramolecular interactions different from the conventional Watson-Crick base pairing with the proximate bases, the so called Hoogsteen base pairing. This reduces the viscous drag of the DNA fragment during electrophoresis.

Previous experiments addressing the resolution of band compressions aimed at a reduction or elimination of intramolecular base-pairing through the full replacement of dGTP with such nucleotide analogues as dITP (Mills D.R. and Kramer, F.R.. Structure-independent Nucleotide Sequence Analysis. *Proc. Natl. Acad. Sci. U.S.A.* **76** (1979), 2232-2235) or 7-deaza dGTP (Mizusawa S., Nishinmuar, S. and Seela, F.. Improvement of the dideoxy chain termination method of DNA sequencing by use of deoxy-7-deazaguanosine triphosphate in place of dGTP. *Nucleic Acids Res.* 14, (1986), 1319-1324).

More particularly, it is known from the literature that the use of ITP for sequencing GC-rich RNA instead of GTP can reduce band compression (Mills D.R. and Kramer F.R., Structure-independent Nucleotide Sequnce Analysis. *Proc. Natl. Acad. Sci. US:A.* **76**, (1976), 2232-2235). The deoxy-analogue of ITP, dITP, was also used in sequencing GC-rich DNA templates to decrease band compression.

The second nucleotide analogue used instead of dGTP in DNA sequencing in order to minimise band compression is 7-deaza dGTP (Mizusawa, S. Nishinmuar, S. and Seela, F.. Improvement of the dideoxy chain termination method of DNA sequencing by use of deoxy-7-deazaguanosine triphosphate in place of dGTP. *Nucleic Acids Res.* 14, (1986), 1319-1324,; Barr, P.J., Thayer, R.M., Laybourn, P., Najarian, R.C. Seela, F. and Tolan, D.R.. 7-deaza-2'-deoxyguanosine-5'-triphosphate: Enhanced Resolution in M13 Dideoxy Sequencing. *Biotechniques* **4**, (1986), 428-432). Guanine and 7-deaza guanine form the same three Watson-Crick hydrogen bonds with cytosine and the strength of the Waston-Crick base pair bond of both analogs are almost identical (Seela, F. et al. (1982)). Thus the reduction of band compression upon use of 7-deaza dGTP can not be due to a decrease in the formation of intramolecular base pairs stabilized by Watson-Crick base pairing.

Despite band compression could be reduced to a certain extent making use of said to deoxy analogues, the performance of such sequencing methods is not appropriate to fulfill the needs of today's sequencing projects, particularly when it comes to the sequencing and amplification of GC-rich nucleic acid sequences.

Accordingly, one aspect of the problem underlying the present invention was to provide a means to allow for increased resolution of band compressions when sequencing or amplifying nucleic acid sequences, particularly GC-rich nucleic acid sequences. Another aspect was to provide means for the sequencing of nucleic acid sequences which reduces the ambiguities during base calling of a sequencing gel. In a further aspect said means should allow for a long average reading length of sequencing gels.

In a still further aspect of the invention a method was to be provided which allows for the sequencing of a nucleic acid sequence avoiding band compressions and allowing more reliable base calling.

This problem is solved by a mixture comprising 7-deaza dGTP or a functional analogue thereof and dITP.

Furthermore a combination comprising the inventive mixture and (NH₄)₂SO₄ is suitable to solve this problem.

Said problem is also solved by a sequencing mixture comprising a mixture according to the invention and/or a combination according to this invention. The problem is also solved by a method for sequencing and/or amplification of a nucleic sequence comprising the steps of preparing a sequence mixture according to the invention which further comprises a nucleic sequence to be sequenced and/or amplified and some enzymatic activity for incorporating dNTPs and/or ddNTPs or derivatives thereof.

Finally, the problem is solved by a sequence gel obtainable by a method according to the invention.

The problem underlying the present invention is also solved by the use of a mixture of the present invention, a combination according to the present invention or a sequencing mixture according to the present invention for sequencing and/or amplification of a nucleic acid sequence.

Further embodiments of the present invention can be taken from the claims the disclosure of which is herewith incorporated by reference.

In a preferred embodiment of the inventive mixture the ratio of 7-deaza dGTP or a functional analogue thereof to dITP is about 200 to 1 to about 1 to 200.

In a more preferred embodiment the ratio of 7-deaza dGTP or a functional analogue thereof to dITP is 4 to 1.

In an alternative preferred embodiment the ratio of 7-deaza dGTP or a functional analogue thereof to dITP is 1 to 4.

In a further embodiment of the inventive mixture said mixture further comprises at least one compound selected from the group comprising dATP, dCTP, dTTP, ddNTPs and derivatives thereof and mixtures thereof.

In a further preferred embodiment the concentration of dATP, dCTP and/or dTTP is each about 100 µM to 4 mM and/or the total amount of 7-deaza dGTP or a functional derivative thereof and dITP is about 100 µM to 4 mM and/or the amount of ddNTPs is about 0.5 to 10 µM.

In a preferred embodiment of the inventive combination (NH₄)₂SO₄ is comprised in a buffer solution.

In a preferred embodiment the concentration of (NH₄)₂SO₄ is about 15 mM.

In an embodiment of the inventive sequencing mixture said sequencing mixture is further diluted.

According to the present invention the inventive mixture, the inventive combination and/or the inventive sequencing mixture can be used for sequencing and/or amplification of a nucleic acid sequence.

In a embodiment of the inventive method the sequencing mixture further comprises at least one primer and an enzymatic activity for amplification of the sequence to be sequenced or amplified.

In a further embodiment of the inventive method said method further comprises the steps of denaturing the at least partially double stranded nucleic acid, annealing the primer to a single stranded nucleic acid and synthesizing nucleotides and/or nucleotide analogues or derivatives thereof to the primer attached to a single stranded nucleic acid.

In a further preferred embodiment of the inventive method said method further comprises applying the sequence reaction products obtainable from any of the aforementioned steps, to a sequencing gel or other detection device capable of reading the sequencing ladder.

Finally, according to the inventive method a further step of reading out the sequencing gel can be comprised.

Also, the present invention relates to a sequence gel obtainable by the inventive method.

Also it is disclosed a sequence gel having less compressed regions than a sequence gel which can be generated upon using 7-deaza dGTP instead of dGTP.

It has surprisingly been found that the combination of 7-deaza dGTP or a functional analogue thereof and dITP allows for a further reduction of band compression compared to a situation where only one of said deoxynucleotides is used. Due to this reduced band compression also a more reliable base calling can be assured. A further advantage resides in the fact that also maximum reading length of a sequence gel can be realised.

Without wishing to be bound by theory it appears that the particular combination of said deoxynucleotide results from a surprising synergistic effect of the mode of action of each single deoxynucleotide. In connection with ITP and dITP, respectively, the mode of action which creates its beneficial effect is said to reside in the replacement of the guanine residue by inosine is in both cases due to the fact that inosine-cytosine base-pairing is characterised through the formation of only two Watson - Crick hydrogen bondings, which are less stable than the original GC-bondings (Barr, P.J., Thayer, R.M., Laybourn, P., Najarian, R.C. Seela, F. and Tolan, D.R.. 7-deaza-2'-deoxyguanosine-5'-triphosphate: Enhanced Resolution in M13 Dideoxy Sequencing. *Biotechniques* **4**, (1986), 428-432). This results in a substantial reduction in the formation of intramolecular "hairpin" type structures stabilized by Watson-Crick base pairing and produces a more uniform size distribution of the DNA in the electrophoresis gel.

In case of 7-deaza dGTP it has been postulated that the inability of 7-deaza dGTP to undergo Hoogsteen base pairing due to the replaced nitrogen at position 7 is the reason for the decrease in GC compressions.

Another possible cause of stable secondary structures are the vertical base-stacking interactions of nucleic acid helices (Saenger, W. . Principles of Nucleic acid structure. C.R. Cantor (Hrsg.), 1984, Springer-Verlag, New York). It is known that a stable single strand helical structure can be produced with polynucleotides made up of only purines. Analogous polynucleotides made up of pyrimidines give rise to a unstacked structure. The major force for the stabilization of base stacks are the dipole interactions of the adjacent bases. The 7-deaza analogue of guanine has an altered electron system and hence a decreased dipole moment compared to guanine. Because of the weaker dipole-dipole interaction intramolecular stacking should be less energetically favored for polynucleotides containing the 7-deaza analogue.

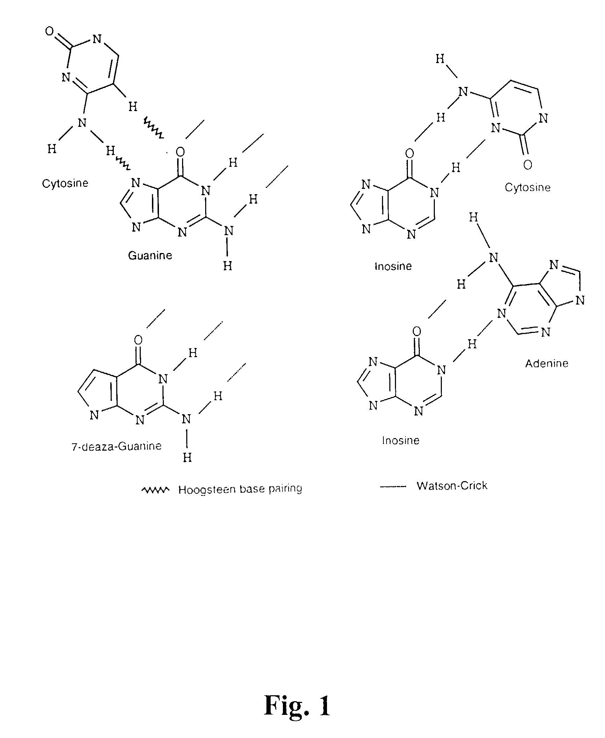

Fig. 1 illustrates these aspects. More particularly, the replacement of the nitrogen at position 7 with a carbon for the 7-deaza form of guanine makes Hoogsteen base pairs impossible. Furthermore, Watson-Crick base pairing between inosine and cytosine is relatively week, because inosine forms only two hydrogen bonds with either cytosine or adenine residues. The effects of 7-deaza dGTP and dITP result in a reduction of intramolecular hairpin structures and thus resolve band compressions in cycle sequencing.

In connection with this the term functional analogue of 7-deaza GTP is to be understood as a chemical compound which allows for the incorporation into a nucleotide sequence whilst allowing for Watson-Crick base pairing and making Hoogsteen base pairs impossible as can be taken, in principle, from Fig. 1. Based on these considerations the one skilled in the art will select an appropriate compound which will then serve as a functional analogue of 7-deaza dGTP.

It has been discovered by the inventors of the present application that from these mode of actions there seem to be a synergistic effect which creates the above-specified advantages in the sequencing and amplification of DNA sequences, and more particularly of GC related nucleic acid sequences.

What is most interesting to note is the fact that particularly a ratio of 7-deaza dGTP and dITP of 4 to 1 and such a ratio of 1 to 4 resulted in optimum resolution of compression while at the same time providing for a maximum reading length.

In connection with this finding of a synergistic effect of a combination of 7-deaza dGTP or a functional analogue thereof and dITP also the buffer used for the sequencing comprising (NH₄)₂SO₄ is to be understood as an important agent. The final concentration of the (NH₄)₂SO₄ containing buffer which is added to the inventive mixture forming the inventive sequencing mixture amounts to a concentration range between 100 and 200 mM, more preferably 150 mM, with the other compounds of said (NH₄)₂SO₄ containing buffer being Tris-HCl pH (9,3) and MgCl₂ with said compounds being comprised in the buffer at a concentration of about 125 mM and 50 mM, respectively, and being further diluted as will be understood by the one skilled in the art when applied to, e. g., a sequencing gel.

In connection with the inventive method it has to be noted that the enzymatic activity incorporating ddNTPs comprises, among others, some DNA polymerase activity. In some preferred embodiments of the inventive method the enzymatic activity comprises enzymes which allow a polymerase chain reaction (PCR) for the amplification of the DNA sequence to be sequenced. The way such sequencing is done is known to the one skilled in the art.

The sequencing gel to be used in the inventive method is preferably electrophoresis gel, and most preferably a polyacrylamide gel. Such gels might be selected from the group comprising countinous and discontinous gels.

If is to be noted that instead of the ddNTPs as mentioned herein, known to act as chain terminators, also derivatives and analogues thereof can be used as long as they still perform chain termination. For example, such derivatives may be those compounds exhibiting an amino group instead of the 2' and 3' deoxy group, respectively.

The concentration ranges of the dNTPs as well as of the ddNTPs and their derivatives, respectively, will depend on the particular enzymatic activity used. For example, in case wild-type Tag polymerase is used, the concentration of ddNTPs is more in the mM range, whereas commercially available designed Tag polymerase such as the one used in the example herein, rather requires concentrations in the µM range.

Furthermore, besides the DNA polymerase activity used in the inventive method, a pyrophosphatase, more preferably a thermostabile pyrophosphatase, can be added to the sequencing mixture.

Finally, it is to be understood that the mixture, combination and sequencing mixture according to the present invention can also be used in a method for direct exponential amplification and sequencing (DEXAS) as described by Kiler et al. (Kilger, C.; Nucl. Acid. Research 1997).

The present invention is further illustrated by the following example and the drawings which provide further embodiments and advantages of the present invention wherein
Fig. 1 is a schematic representation showing hydrogen bonding in case of Watson-Crick and Hoogsteen base pairing between guanine and cytosine residues in single stranded DNA;
Fig. 2 shows a DNA sequence of compression prone regions and their resolution;
Fig. 3 shows gel and trace images depicting the resolution of a compressed region in comparison with a non-compressed region which has been resolved using the inventive mixture and method, respectively; and
Fig. 4 shows average reading length in dependence on the amount of 7-deaza dGTP in the mix with dITP.

The effect of using a combination of 7-deaza dGTP and dITP instead of dGTP was evaluated for the Dye-Primer cycle sequencing method. We have chosen 4 templates stemming from ongoing genome projects for the analysis. The base contents of all four templates show a high percentage on guanine and cytosine residues. For template A the GC content is 59 %, for template B 58.3% GC, for template C 57.5 % GC, and for template D 49.7 % GC. In comparison *E.coli* has a GC content of 50,8 %. The doublex sequencing reactions were carried out using 1µg plasmid DNA with a 2000 bp insert, 2 pmol of each upstream and downstream Primer, 10 U AmpliTaq FS, 2µl buffer (containing 125 mM Tris-HCl pH (9.3), 150 mM (NH₄)₂SO₄, 50 mM MgCl₂) in a total volume of 20 µl. 5 µl of this reaction mixture were individually combined with 2 µl of the nucleotide mixtures containing each 1 mM dATP, dCTP, dTTP and combinations of 7-deaza dGTP and dITP in ratios varying from 1 to 0.1 in steps of 10% at a constant total dITP / 7-deaza dGTP concentration of 2 mM. Each mixture was supplemented with one of the four ddNTPs at a concentration of 2,5 µM. When using 100 % 7-deaza dGTP in the nucleotide mixture instead of dGTP, the buffer conditions were changed to the commercially available lx sequencing buffer from Perkin Elmer (Perkin-Elmer, Foster City, California) containing 50 mM Tris-HCl pH 8.9, 10 mM KCl, 2.5 mM MgCl₂, 0.25 % Tween^{R} 20. All reactions were run under identical cycling conditions applying 30 cycles consisting of 15 s at 97°C (denaturing), 30 s at 55°C (annealing) and 120 s at 68°C (synthesis). The sequencing reaction products were separated on the same electrophoresis gel on a LICOR DNA-Sequencer *Long READIR* 4200 (LI-COR *Biotechnology Division;* Lincoln, Nebraska, USA).

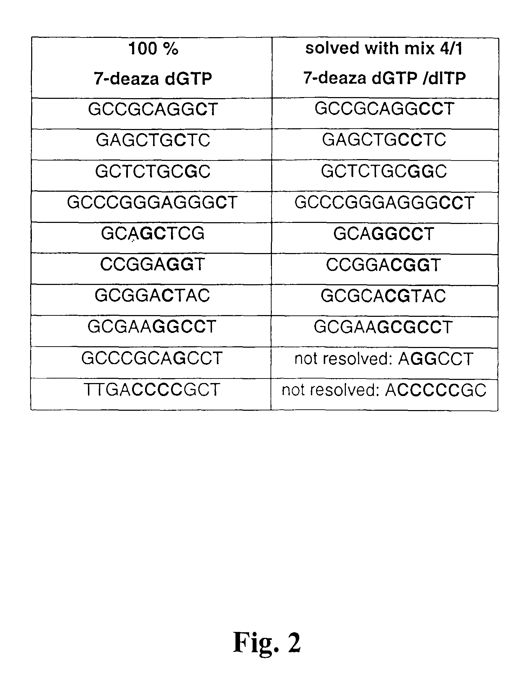

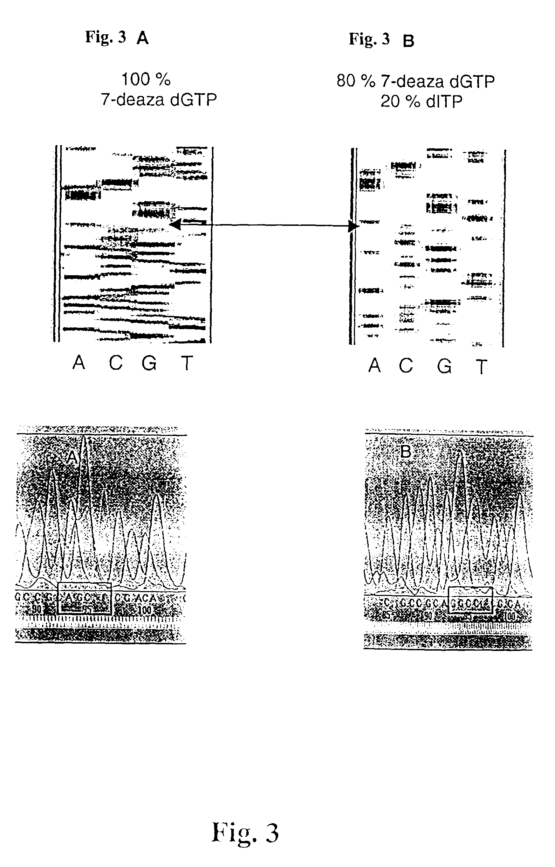

While sequencing the templates with the reagents containing 100 % 7-deaza dGTP in the nucleotide mixture we found 10 compressed regions. The surrounding of 8 to 12 bases of the compressed regions are characterized by a 66 % to 85 % content of guanine and cytosine residues (Fig. 2). As a result 80% of the band compressions could be resolved using the nucleotide mixture 7-deaza dGTP and dITP at a ratio of 4. In all cases with the DNA sequence GCCT one cytosine in the base assignment was skipped when sequencing was performed with 7-deaza dGTP. The other DNA segments displayed deviant base composition, but altogether in 6 out of 8 cases a cytosine was skipped upon base calling. Base calling of the sequencing reactions of these DNA segments after electrophoresis skipped a cytosine residue in 75 % of the examples. We have accordingly modified sequencing reactions of these templates by varying the nucleotide constitution. We totally replaced dGTP by a mixture of 7-deaza dGTP and dITP, employing varying ratios of both nucleotide analogues from 1 to 0.1 in steps of 10 %. Our observation was that for 8 of the 10 observed compressed regions the sequence could be processed correctly with the nucleotide mixture containing a 7-deaza dGTP / dITP quotient of 4, the mixture containing a 7-deaza dGTP / dITP ratio of 0.25 interestingly also led to a resolution of 7 of these compressed regions. The nucleotide mixture containing both analogues at equal rates showed a resolution of only 4 of these compressed regions, the other ratios applied to the reaction did not lead to a further resolution of the band compressions compared to the 7-deaza dGTP / dITP ratio of 0.25 or 4, which is shown in figure 3 as one example of the resolution of a band compression. More particulary, Panel A shows the sequencing ladder obtained upon employing the 100 % 7-deaza dGTP in the sequencing reaction within the commercial available buffer form Perkin-Elmer. For Panel B the sequencing ladder was produced by using a nucleotide mixture of 80 % 7-deaza dGTP and 20 % dITP, which means a ratio of 4 to 1, with a (NH₄)₂SO₄ containing buffer. The trace files in panel B depict the decompressed sequence, AGGCCT, whereas in panel A the signs of the polynucleotide ending with a cytosine is located at the same position as the polynucleotide ending up with the previous guanine. This band compression leads to a wrong sequence-read, AGCT.

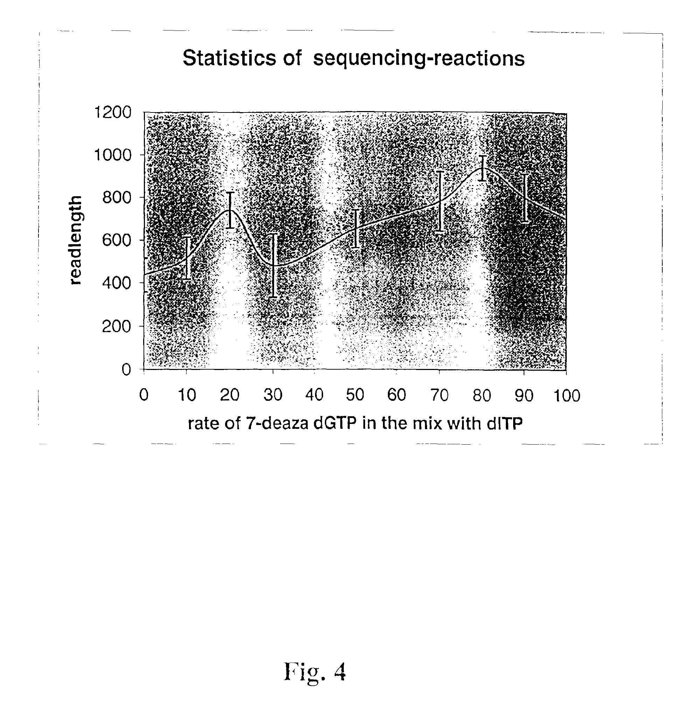

Since it was desirable to combine optimal resolution of compressions with maximum reading length, the latter parameter was analyzed in dependence from the ratios of employed nucleotide analogs as well. Interestingly two maximums of reading length were observed at a 4 to 1 ratio and the vice versa ratio of the combination of 7-deaza dGTP and dITP applied to the sequencing reaction (Fig. 4). As can be taken from Fig. 4, each data point in this diagram represents the average reading length of 16 single sequencing reactions having less than 2% ambiguity. The sequencing reactions were carried out with four templates and the upstream and revers primer using a titration of nucleotide ratios of 7-deaza dGTP and dITP in combination with a (NH₄)₂SO₄-buffer. The average reading length in case of the 4/1 deaza- dGTP / dITP mixture turned out to be 100 bases longer than with the 1/4 ratio of both nucleotide analogues. Shorter reading lengths were obtained using intermediate ratios of both nucleotide analogues. A typical sequencing reaction resolving compressions due to the use of the 4/1 ratio of 7-deaza dGTP/dITP was a read of 1156 bases with 8 ambiguities while calling a total of 1450 bases with 68 ambiguities.

The features disclosed in the foregoing description, in the claims and/or in the accompanying drawings may, both separately and in any combination thereof, be material for realising the invention in diverse forms thereof.

## Claims

1. Mixture comprising 7-deaza dGTP or a functional analogue thereof and dITP.

2. Mixture according to claim 1 wherein the ratio of 7-deaza dGTP or a functional analogue thereof to dITP is about 200 to 1 to about 1 to 200.

3. Mixture according to claim 2, wherein the ratio of 7-deaza dGTP or a functional analogue thereof to dITP is 4 to 1.

4. Mixture according to claim 2, wherein the ratio of 7-deaza dGTP or a functional analogue thereof to dITP is 1 to 4.

5. Mixture according to any of claims 1 to 4 further comprising at least one compound selected from the group comprising dATP, dCTP, dTTP, ddNTPs and derivatives thereof and mixtures thereof.

6. Mixture according to any of claims 1 to 5 wherein the concentration of dATP, dCTP and/or dTTP is each about 100 µM to 4 mM and/or the total amount of 7-deaza dGTP or a functional derivative thereof and dITP is about 100 µM to 4 mM and/or the amount of ddNTPs is about 0.5 to 10 µM.

7. Combination comprising the mixture according to any of claims 1 to 6 and (NH₄)₂SO₄.

8. Combination according to claim 7 wherein (NH₄)₂SO₄ is comprised in a buffer solution.

9. Combination according to claim 7 or 8 wherein the concentration of (NH₄)₂SO₄ is about 15 mM.

10. Sequencing mixture comprising a mixture according to any of claims 1 to 6 and/or a combination according to any of claims 7 to 9.

11. Sequencing mixture according to claim 10 wherein the sequencing mixture is further diluted.

12. Use of a mixture according to any of claims 1 to 6 or of a combination according to any of claims 7 to 9 or a sequencing mixture according to claim 10 or 11 for sequencing and/or amplification of a nucleic acid sequence.

13. Method for sequencing and/or amplification of a nucleic acid sequence comprising the steps of
- preparing a sequencing mixture according to claim 10 or 11 which further comprises a nucleic acid sequence to be sequenced and/or amplified and some enzymatic activity for incorporating dNTPs and/or ddNTPs or derivatives therof.

14. Method according to claim 13, wherein the sequence mixture further comprises at least one primer and an enzymatic activity for amplification of the sequence to be sequenced or amplified.

15. Method according to claim 13 or 14 further comprising the steps of denaturing the at least partially double stranded nucleic acid, annealing the primer to a single stranded nucleic acid and synthesizing nucleotides and/or nucleotide analogues or derivatives thereof to the primer attached to a single stranded nucleic acid.

16. Method according to any of claims 13 to 15 further comprising the step of applying the sequence reaction products obtainable from any of the aforementioned steps, to a sequencing gel or other detection device capable of reading the sequencing ladder.

17. Method according to any of claims 13 to 16 further comprising the step of reading out the sequencing gel.

18. A sequence gel obtainable by a method according to any of claims 13 to 17.

19. A sequence gel having less compressed regions than a sequence gel which can be generated upon using 7-deaza dGTP instead of dGTP.
